# FASCICULE DE BREVET EUROPEEN

(11) **EP 4 255 355 B1**
(45) Date de publication et mention de la délivrance du brevet: **08.01.2025**
(21) Numéro de dépôt: 21840648.6
(22) Date de dépôt: 30.11.2021
(51) Int. Cl.: A61F 2/46

(54) **DISPOSITIF DE PRÉHENSION D'UN ÉLÉMENT PROTHÉTIQUE TELLE UNE CUPULE ET PROCÉDÉ D'UTILISATION D'UN DISPOSITIF DE PRÉHENSION COMME INSERT D'ESSAI**
VORRICHTUNG ZUM GREIFEN EINES PROTHESENELEMENTS WIE ETWA EINES BECHERS UND VERFAHREN ZUR VERWENDUNG EINER GREIFVORRICHTUNG ALS VERSUCHSEINSATZ
DEVICE FOR GRIPPING A PROSTHETIC ELEMENT SUCH AS A CUP, AND METHOD FOR USING A GRIPPING DEVICE AS TRIAL INSERT

(30) Priorité: 01.12.2020 FR 2012490; 01.12.2020 FR 2012492
(43) Date de publication de la demande: 11.10.2023
(73) Titulaire: Majou, Claude, 01120 Montluel (FR); Manin, Christian, 69150 Decines (FR)
(72) Inventeur: Majou, Claude, 01120 Montluel (FR); Manin, Christian, 69150 Decines (FR)
(74) Mandataire: Leone, Eloïse
(86) Numéro de dépôt international: PCT/FR2021/052152
(87) Numéro de publication internationale: WO 2022/117949

(56) Documents cités:
- EP-A1- 1 438 936
- WO-A1-2018/109379
- FR-A1- 3 066 908
- US-A1- 2013 304 072

## Description

### DOMAINE D'APPLICATION DE L'INVENTION

La présente invention se rapporte à un dispositif de préhension d'éléments prothétiques telles des cupules et notamment aux adaptations permettant d'assurer cette préhension dans les meileures conditions et d'adapter cette préhension afin de simplifier les phases de tests peropératoires. L'art antérieur le plus proche est le document WO 2018/109371 A1, qui définit le préambule de la revendication 1.

### DESCRIPTION DE L'ART ANTÉRIEUR

Les prothèses cotyloïdiennes sont bien connues dans le domaine orthopédique. Elles sont composées d'une cupule métallique de forme extérieure plus ou moins hémisphérique. prolongée parfois d'un cylindre tronqué ou entier.

La cupule présente une surface externe qui dépasse ou non l'équateur, une surface interne concave hémisphérique continue ou partiellement conique, dépourvue ou non de trou polaire fileté pour la connexion d'un instrument.

Cette cupule peut être une cupule à double mobilité avec une surface interne hémisphérique, lisse, polie, ou une cupule à simple mobilité dont la surface interne comporte un cône de coincement avec une rugosité spécifique, prolongé de calottes plus ou moins sphériques. Ainsi, l'intérieur de la cupule peut être de forme variable : hémisphérique, hémisphérique prolongée d'un cylindre, partielement conique.

Cette cupule est destinée à être implantée dans le cotyte osseux d'un patient Un insert est ensuite assemblé à l'intérieur de la cupule formant l'articulation dans laquelle prend place une tête prothétique fémorale.

Les états de surfaces intérieures peuvent varier : pour un couple de frottement métal/polyéthylène, il est poli, pour un couple avec un insert céramique, la surface conique présente une rugosité adaptée au montage.

Après avoir préparé le site osseux des tests peropératoires dynamiques sont effectués pour vérifier l'adéquation des tailles de cupules avec des positions (longueurs et diamètres) de têtes associées aux tiges fémorales. Ces tests sont réalisés avec des pièces d'essais mises à disposition dans l'ancillaire. Les tests renseignent la configuration optimale (longueur du membre, stabilité articulaire, amplitudes de mouvements) des composants à implanter. les cupules métalliques définitives sont orientées et installées dans le cotyle osseux du patient l'insert polyéthylène ou céramique est assemblé in-situ, la tête fémorale prothétique de longueur adaptée est montée sur la tige fémorale puis l'articulation est réduite.

Pour lier les cupules à un instrument afin de les transporter, de les positionner et de les libérer dans le patient, il existe actuellement plusieurs solutions techniques,

Il existe par exemple des systèmes qui se vissent au fond des cupules. Ceci n'est pas possible dans certaines configurations et lorsque cela est possible, il est conseifié d'obstruer le trou fileté pour éviter les migrations de participes d'usure, ce qui est coûteux en temps de manipulation. En effet, la mise en oeuvra d'un tel système requiert le vissage et la dévissage de instrument, le vissage d'un bouchon obturateur.

Un autre système décrit dans le document US10,245, 161 consiste à se lier temporairement à la cupule. A cette fin, ce document décrit un dispositif de préhension et de pose d'une cupule cotyloïdienne comportant au moins deux rebords enctiquetables. Le dispositif comprend un préhenseur qui est un sous-ensemble séparé de la poignée. Ce préhenseur comprend des dents aptes à s'engager avec les rebords de la cupule cotyloïdienne. Le préhenseur comprend un poussoir mobile, capable, lors de son mouvement, d'exercer une poussée sur la cupule cotyloïdienne le long de raxe de révolution de ladite cupule cotyloïdienne, de manière à dégager les rebords de la cupule cotyloïdienne de leur engagement avec les dents incluses par le préhenseur. Le dispositif de préhension comprend un dispositif d'actionnement pour actionner la poussoir mobile,

Un tel dispositif présente plusieurs inconvénients décrits ci-après.

En raison des différentes surfaces en opposition la qualité de la préhension est limitée. De plus, la transmission des forces mises en action sur des zones sans adhérence de la cupule n'est pas efficace lors des sollicitations et conflits engendrés pendant l'installation de l'implant.

La manipulation de cette cupule est rendue difficile par des voies d'abord chirurgicales indirectes qui contournent des tissus mous et osseux, des incisions très courtes,

De plus, avec les instruments actuels de préhension, il existe le risque de désolidarisation de la cupule avec le préhenseur lors de la mise en place dans et au fond du site osseux En effet l'adhérence dépend du nombre de dents en prise avec les rebords enclquetables. Cela peut induire une imprécision de l'implantation, un endommagement de l'os, du temps supplémentaire pour conduire l'intervention.

L'utilisation d'une interface stérile montée préalablement en laboratoire sur la cupule existe (comme le décrit également US10.245.161). Il présente l'avantage de pouvoir être relié rapidement à un dispositif de préhension sans être contaminé par aucune manipulation extérieure. La sécurité du montage en laboratoire est contrôlée. Cependant la pièce intermédiaire doit être usinée d'une façon très rigoureuse et contraignante, ce qui induit des coûts très élevés. De plus, l'obtention par moulage n'est pas envisageable car le matériau utilisé doit également répondre aux exigences de biocompatibilité ce qui limite le choix ou augmente considérablement l'argumentation médico-technique à développer.

Enfin, pour la solution décrite dans US10.245.161. les différents éléments doivent être positionnés précisément de façon à pouvoir mettre en œuvre leur fonction. En effet, les dents doivent être positionnées sur les parties encliquetables. De plus, le préhenseur doit être bien orienté dans la pièce intermédiaire.

Un autre inconvénient concerne la phase de tests peropératoires décrite plus haut Pour réaliser les tests de longueur du membre en peropératoire, il est nécessaire de disposer de toutes les versions dans toutes les tailles d'inserts d'essai. En fait, l'ensemble des composants de la prothèse définitive est reproduit par les pièces d'essais. Toutes ces pièces d'essai représentent un volume très important dans les boites ancillaires dédiées ainsi qu'un coût pour la reconditionnement et/ou la stérilisation après chaque utilisation. Avec les instruments actuels d'essai de longueur de membre, il est nécessaire de nettoyer le fond de la cupule pour y installer un insert d'essai dont il faut bien identifier le type et la taile ainsi que le diamètre de la tête d'essai, il existe un risque d'indisponibilité et d'erreur de présentation au chirurgien.

Un autre inconvénient réside dans le fait que les instruments proposés pour lier rapidement les cupules sont des mécanismes enfermés qui ne sont pas totalement démontables. Ils sont alors difficiles d'accès pour un nettoyage complet et efficace.

### BRÈVE DESCRIPTION DE L'INVENTION

Ce que constatant les demandeurs ont mené des recherches visant à proposer une connexion fiable, rapide et simple d'un élément prothétique telle une cupule prothétique sur un outil de préhension destiné à la transporter, à la positionner et à la libérer sur le site osseux du patient

Un tel dispositif est destiné à la préhension d'implant stériles ou non stériles, de composants prothétiques, cotyloïdiens. glénoïdiens, de pièces d'essai, cupules d'essai, inserts d'essai.

Ces recherches ont abouti à la conception et à la réalisation d'un dispositif de préhension pour élément prothétique telle une cupule,
l'élément prothétique comprenant un volume intérieur présentant une surface intérieure,
le dispositif comprenant un outil de préhension coopérant ponctuellement avec l'êlément prothétique à des fins de manipulation et/ou d'installation sur le patient
ledit outil comprenant un manche avec à une extrémité, une poignée et à une autre extrémité, un module d'agrippement,
ledit module d'agrippement étant commandé par raction de l'utilisateur sur une lame levier associée au manche pour passer d'une position de retenue à une position de repos et vice-versa.

Ce dispositif est remarquable en ce qu'il comprend une interface mécanique s'intercalant entre l'élément prothétique et le module d'agrippement et venant se fixer à l'élément prothétique,
ladite interface mécanique présentant une surface extérieure convexe se projetant en partie dans le volume intérieur de l'élèment prothétique et épousant la surface intérieure de l'élément prothétique, et un volume intérieur concave avec lequel coopère le module d'agrippement,
l'interface mécanique se composant d'une pluralité de lamelles qui, rayonnant autour d'une même partie centrale, adoptent un profil avec une surface interne et une surface externe, ladite surface externe forme ladite surface extérieure convexe pour épouser tout ou partie du profil de la surface intérieure de l'élément prothétique,
ladite interface mécanique étant préformée et réalisée dans un matériau tel que son élasticité permet sa retenue dans l'élément prothétique,
de sorte que
   - la position de repos du module d'agrippement commandée parla lame levier autorise ce dernier à venir s'introduire dans le volume intérieur concave et à sortir de ce dernier, et que
   - la position de retenue permet au module d'agrippement introduit dans le volume intérieur concave d'agripper l'interface et de retenir l'élément prothétique lorsque ce dernier doit être déplacé ou de libérer l'interface mécanique de l'élément prothétique lorsque ce dernier est en place et fixé à l'os du patient,
le volume intérieur concave de l'interface mécanique étant préformé de surfaces périphériques d'appui avec lesquelles coopèrent des surfaces de retenue périphériques préformées dans le module d'agrippement de sorte qu'un mouvement commandé par la lame levier assure la création d'une contrainte tendant à étendre les surfaces de retenue périphériques de façon à assurer la fixation ponctuelle du module d'agrippement à l'interface mécanique.
ledit module d'agrippement comprend une couronne d'agrippement équipée extérieurement desdites surfaces de retenue périphériques et intérieurement de surfaces inclinées périphériques intérieures coopérant avec des surfaces inclinées périphériques extérieures ménagées sur la surface extérieure d'un arbre d'expansion mobile en translation et dont une extrémité est articulée à la lame levier de sorte que l'action sur la lame levier provoque un mouvement en translation amenant à l'expansion des surfaces de retenue périphériques ou leur rétractation en position de repos.

L'interface mécanique peut être :
- montée/assemblée puis stérilisée avec l'élément prothétique,
- disponible dans la bole ancillaire sur la table opératoire,
- désassemblée et repositionnée in-situ.

L'utilisation d'une interface mécanique venant s'introduire dans le volume intèrieur d'un élément prothétique telle une cupule est particulièrement avantageuse en ce qu'elle augmente en épousant ledit volume intérieur, la surface d'adhérence à des fins de préhension sans rendre plus volumineux l'ensemble obtenu.

Il permet en outre de séparer deux parties : une partie dédiée à l'élément prothétique et à ses dimensions avec la surface extérieure de l'interface et une partie destinée à la coopération avec l'outil de préhension et son module d'agrippement. Cette dernière partie peut être identique quelles que soient les dimensions de l'élément prothétique de sorte que le module d'agrippement puisse être commun pour toute dimension d'élément prothétique,

La configuration en lamelles rayonnantes apporte l'élasticité nécessaire à l'interface afin de permettre son maintien en position dans l'élément prothétique. La configuration en lamelles rayonnantes apporte l'élasticité nécessaire à l'interface afin de permettre son maintien en position dans l'élément prothétique.

La configuration des lamelles rayonnantes intègre les secteurs de contrainte qui transmettent l'effort sur plusieurs surfaces de contact congruent dans la zone équatoriale interne de l'élément prothétique. Le découpage en portions angulaires favorise et permet les jeux fonctionnels nécessaires à la réalisation par moulage de l'interface mécanique. Le nombre des lamelles contribue à contenir les volumes des éléments de l'axe d'expansion.

Selon une autre caractéristique particulièrement avantageuse de l'invention, la surface extérieure convexe formée parla surface externe desdites lameles, est préformée d'une gorge périphérique ménagée entre une partie de surface convexe se positionnant au fond du volume intérieur de l'élément prothétique et une partie de surface convexe se positionnant au niveau du rebord de l'élément prothétique. Cette gorge constitue une séparation dans le profil extérieur des lamelles contribuant à leur élasticité,

Selon une autre caractènstique particulièrement avantageuse de l'invention, la surface interne du profil desdites lamelles est préformée desdites surfaces périphériques d'appui.

Selon une autre caractéristique particulièrement avantageuse de l'invention, ledit module d'agrippement comprend une tête du module d'agrippement qui guide la translation de l'arbre d'expansion, ladite tête étant fendue par une fente. Cette tête est fendue par une fente de façon à faciliter le montage et le démontage avec l'arbre d'expansion lié à la lame levier.

Selon une autre caractéristique particuliérement avantageuse, ladite couronne d'agrippement comprend une fente ouvrant l'anneau qu'elle forme. Cette fente pratiquée dans la couronne d'agrippement participe à la même fonction que la fente pratiquée dans la tête. Le nettoyage complet de l'instrument et le changement de pièces de tailles et dimensions adaptées sont donc facilités.

Selon une autre caractéristique particuliérement avantageuse de l'invention, le matériau de l'interface mécanique est un matériau rigide et biocompatible,

Selon une autre caractéristique particulièrement avantageuse de l'invention, le matériau de l'interface mécanique est de la résine polypropylène, dont une plus spécifique commercialisée sous la nom commercial de propilux.
La résine polypropylène a pour avantage d'être rigide et biocompatible. Il autorise en outre une réalisation par moulage.

Selon une autre caractéristique particuliérement avantageuse de l'invention, l'interface mécanique comporte des dents coopérant avec des surfaces préformées sur le rebord de l'élément prothétique. Ces dents sont optionnelles. Elles Jouent la rôle de butées, êlles s'opposent lorsque l'élément prothétique est une cupule, aux sollicitations en rotation et torsion de la liaison cupüle/module d'agrippement Ces dents assurent la tien entre la cupde et le préhenseur dans le conditionnement stérile. En complément de leur rôle de butée, elles permettent de positionner l'interface mécanique en relation avec des éléments optionnels et complémentaires de la cupule: trous internes, pattes périphériques.

Selon une autre caractéristique particulièrement avantageuse de l'invention, le volume concave de l'interface mécanique est dimensionné et préformé pour présenter des surfaces d'appui correspondant à celes d'un insert destiné à être accueilli après mise en place de l'élément prothétique pour recevoir une tête d'essai ou définitive. Ainsi, l'interface mécanique de l'invention qui facilite la préhension des éléments prothétiques telle une cupule avec les avantages décrits ci-dessus a pour autre fonction de servir également d'insert d'essai. A cette fin, la surface concave hémisphérique située en lieu et place de la tête et qui permet le guidage et le positionnement du module d'agrippement, conditionne le centre de la tête d'essai ou de la tête définitive. L'interface permet de faire directement les tests in-situ sans avoir à utiliser d'autres instruments. Cela sécurise l'association des composants, limite les erreurs de montage et procure un gain de temps opératoire, Lorsqu'il est conditionné avec l'implant, l'interface mécanique remplace les inserts d'essais dans les boîtes d'instruments, libère une place sur la table et élimine le nettoyage et le reconditionnement

Un autre objet de l'invention est donc un procédé d'utilisation d'un dispositif de préhension pour élément prothétique telle une cupule définie par tout ou partie des caractéristiques ci-dessus décrites remarquable en ce qu'il consiste à utiliser ladite interface mécanique comme insert d'essai.

Les concepts fondamentaux de l'invention venant d'être exposés ci-dessus dans leur forme la plus élémentaire, d'autres détails et caractéristiques ressortiront plus clairement à la lecture de la description qui suit et en regard des dessins annexés, donnant à titre d'exemple non limitatif, des modes de réalisation d'un dispositif de préhension de cupule conforme à l'invention.

### BRÈVE DESCRIPTION DES DESSUS

[Fig. 1] est un dessin schématique d'une vue extérieure de côté d'un mode de réalisation d'un dispositif conforme à l'invention ;
[Fig. 2] est un dessin schématique d'une vue en perspective éclatée de l'extrémité de préhension du dispositif de la figure 1 ;
[Fig. 3] est un dessin schématique d'une vue en coupe de l'extrémité de préhension du dispositif de la figure 1 ;
[Fig. 4) et [Fig. 5] sont des dessins schématiques des vues extérieures en perspective de l'accueil d'une tête d'essai ou définitive :
[Fig. 6] est un dessin schématique d'une vue extérieure de côté d'une interface mécanique conforme à l'invention ;
[Fig. 7] est un dessin schématique d'une vue extérieure de dessus de l'interface mécanique de la figure 6 :
[Fig. 8] est un dessin schématique d'une vue extérieure de côté d'une couronne d'agrippemert conforme à l'invention :
[Fig. 9] est un dessin schématique d'une vue extérieure de dessus de la couronne d'agrippement de la figure 8 ;
[Fig. 10] est un dessin schématique d'une vue extérieure de côté d'un arbre d'expansion mobile conforme à l'invention ;
[Fig. 11] est un dessin schématique d'une vue extérieure de dessus de l'arbre d'expansion de la figure 10 ;
[Fig. 12] est un dessin schématique d'une vue extérieure de côté de la tête du manche de l'outil de préhension :
[Fig. 13] est un dessin schématique d'une vue extérieure de dessus de la tête de manche de la figure 12.

### DESCRIPTION D'UN MODE PRÉFÉRÉ DE RÉALISATION

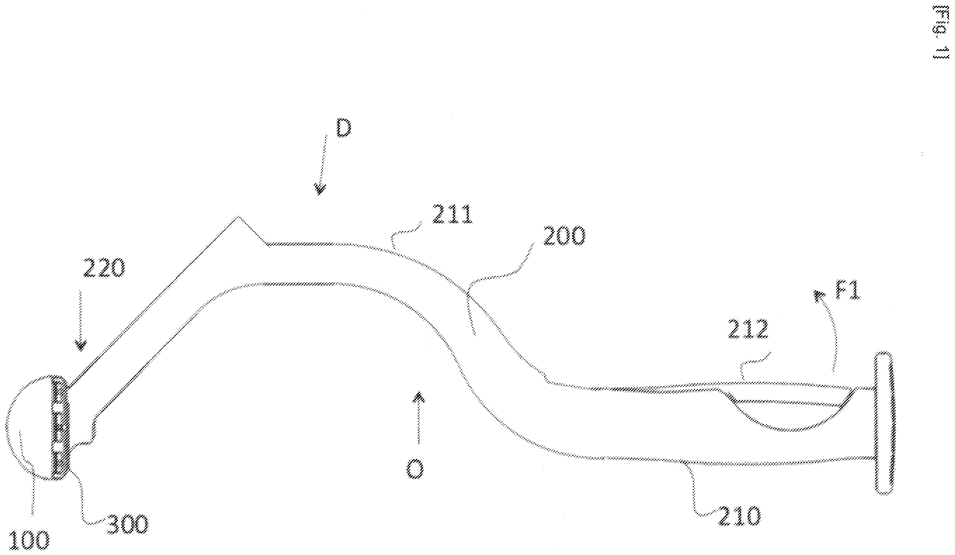

Comme illustré par la figure 1, le dispositif référencé D dans son ensemble assure la préhension d'une cupule 100 au moyen d'un outil de préhension O à des fins de manipulation et/ou d'instalation sur le patient (non illustré).

Cet outil O comprend un manche 200 avec à une extrémité une poignée 210 et à une autre extrémité dite de préhension un module d'agrippement 220.

Ledit module d'agrippement 220 est commandé par l'action de l'utilisateur (non illustré) sur une lame levier 211 associée au manche 200 pour passer d'une position de retenue à une position de repos et vice-versa. L'utilisateur actionne la lame levier 211 en faisant passer le levier pivotant de la position de repos illustrée à une position de travail (non illustrée) par pivotement selon la flèche F1

Comme illustré, le dispositif D comprend une interface mécanique 300 fixée à la cupule 100 avant préhension par l'outil O et avec laquelle coopère le module d'agrippement 220.

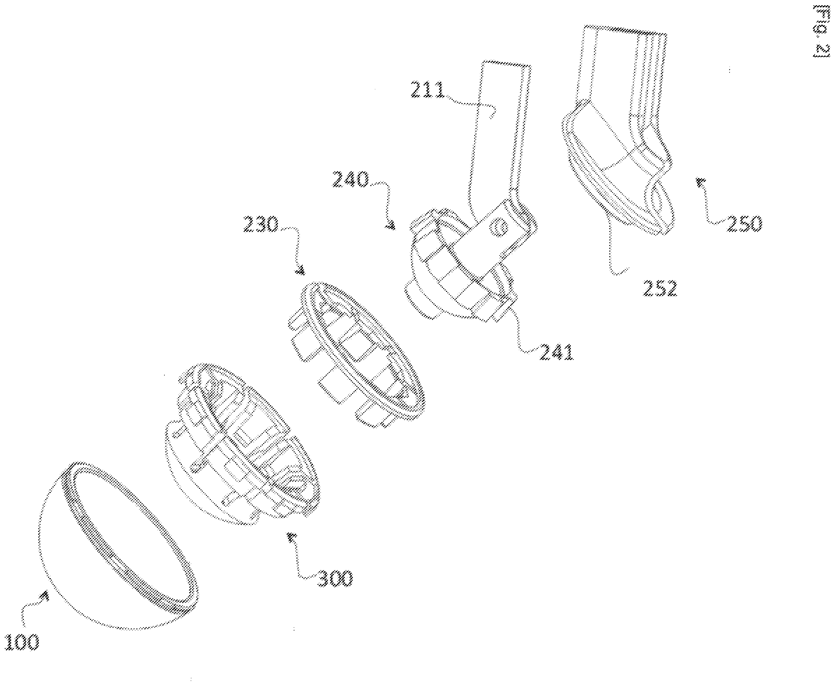

Comme illustrée par les figures 2, 3, 8 et 7. ladite interface mécanique 300 présente une surface extérieure convexe 310 épousant la surface intérieure de la cupule 100 et un volume intérieur concave 320 avec lequel coopère le module d'agrippement 220.

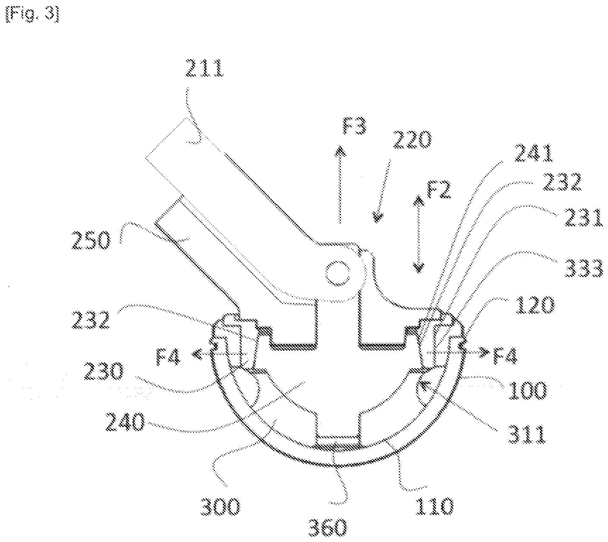

Cette interface mécanique 300 se compose d'une pluralité de lamelles 330 correspondant à des portions angulaires qui, rayonnant autour d'une même partie centrale 340, adoptent un profil avec une surface interne 331 et une surface externe 332. Ladite surface externe 332 forme ladite surface extérieure convexe 310 épousant une partie du profil sensiblement circulaire de la surface < ntèneure 110 de la cupule 100 (cf. figure 3),

Cette interface mécanique 300 présente une élasticité issue de son matériau mais aussi de sa conception, Ainsi, la surface extérieure convexe 310 formée par la surface externe 332 desdites lamelles 330, est préformée d'une gorge périphérique 311 ménagée entre une partie de surface convexe se positionnant au fond de la cupule et une partie de surface convexe se positionnant au niveau du rebord 120 de la cupule 100. Cette gorge 311 adopte un profil demi-circulaire ouvert sur l'extérieur. Une autre caractéristique participant à l'élasticité de l'interface mécanique 300 concerne les fentes 350 séparant les différentes lamelles 330. Cette élasticité permet la retenue de l'interface mécanique 300 dans la cupule 100.

L'interface mécanique comporte en outre sur la périphérie extérieure de son bord supérieur des dents 360 coopérant avec des surfaces préformées à cet effet sur le rebord 120 de la cupule.

La surface interne 331 des lamelles 330 forme le volume intérieur concave 320 de l'interface mécanique 300. Elle est préformée en partie supérieure de surfaces périphériques d'appui 333 tournées vers l'axe central de l'interface mécanique 300.

Comme illustré par les figures 2 et 3. lorsque le module d'agrippement 220 s'introduit dans la volume intérieur concave 320, lesdites surfaces d'appui périphérique 333 se retrouvent en vis-à-vis de surfaces de retenue périphériques 231 préformées dans le module d'agrippement 220.

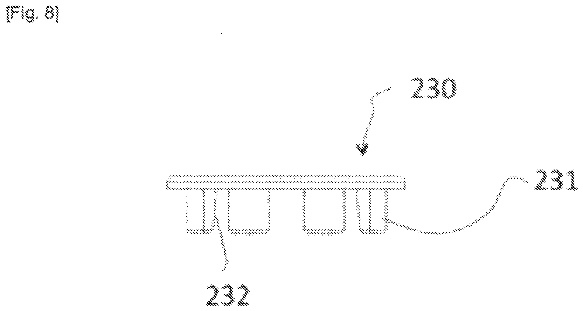

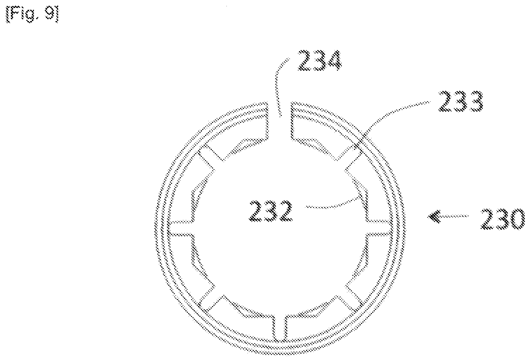

Comme illustrées plus en détail par les figures 8 et 9, ces surfaces de retenue périphériques 231 sont constituées par la surface extérieure de projections se projetant vers le bas à partir d'une couronne d'agrippement 230, élément constitutifs du module d'agrippement 220. Ces projections sont également préformées de surfaces incinées périphériques intérieures 232 tournées vers l'axe central de la couronne d'agrippement 230. Comme illustrée, cette couronne d'agrippement 230 présente une certaine élasticité de par son maténau mais également du fait de sa configuration. Ainsi, les projections sont écartées et séparées par des fentes 233. De plus, l'anneau formé par la couronne 230 est lui-même fendu. Cette fente 234 a une autre fonction qui sera décrite plus loin.

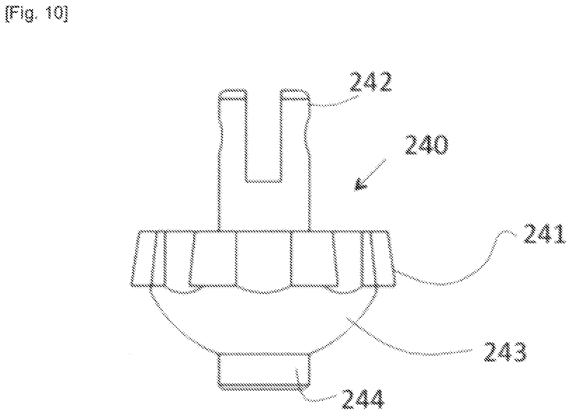

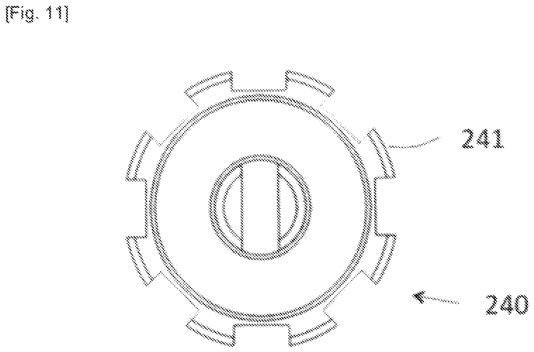

Comme illustrées par les figures 2 et 3. ces surfaces inclinées périphériques intérieures 232 coopèrent avec des surfaces inclinées périphériques extérieures 241 ménagées sur la surface extérieure d'un arbre d'expansion mobile 240 en translation (selon la double-flèche F2) et dont une extrémité 242 est articulée à la lame levier 211 de sorte que le mouvement de la lame levier 211 commandé par la rotation du levier 212 provoque le mouvement en translation de l'arbre 240, Comme illustrées plus en détail par les figures 10 et 11, les surfaces inclinées périphériques extérieures 241 sont préformées sur la périphérie d'un renflement dudit arbre. La partie basse 243 de ce renflement épouse la surface du volume concave. En outre, une projection axiale 244 vient s'introduire dans un orifice axial 370 pratiqué dans le fond de l'interfece mécanique 300 à des fins de positionnement et de guidage de la translation selon la double-flèche F2.

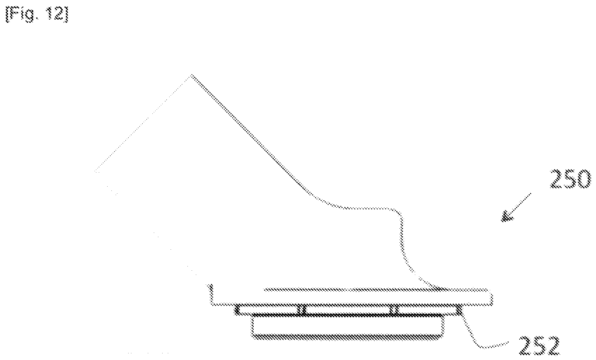

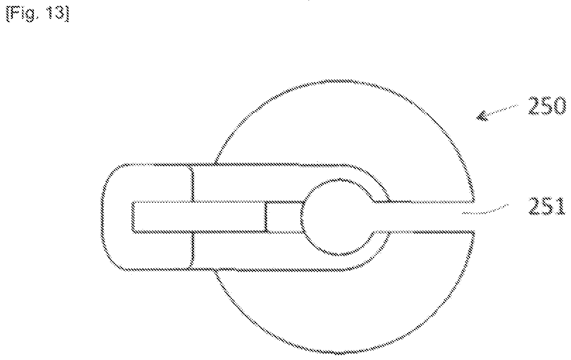

Comme illustré par les figures 2 et 3. un autre élément constitutif du module d'agrippement est constitué par la tête 250 du module d'agrippement 220 qui guide la translation (double-flèche F2) de l'arbre d'expansion 240. Comme illustrée plus en détails par les figures 12 et 13, cette tête 250 est fendue par une fente 251 de façon à faciliter le montage et le démontage avec l'arbre d'expansion 240 lié à la lame levier 211. La fente 234 pratiquée dans la couronne d'agrippement 230 participe à la même fonction. Le nettoyage complet de l'instrument et le changement de pièces de tailles et dimensions adaptées sont donc facilités.

La face inférieure de cette tête 250 est en outre préformée d'une projection hexagonale 252 permettant l'onentabon et le bon positionnement de la couronne d'agrippement 230 préformée à cet effet.

La figure 2 montre la coaxialité des différents éléments : cupule 100, interface mécanique 300 et module d'agrippement 220.

La préhension de l'interface mécanique 300 associée à la cupule 100 par le module d'agrippement 220 est illustrée par la figure 3. Cette préhension peut avoir lieu pour la mise en place de la cupule 100 ou la libération de l'interface mécanique 300 par rapport à la cupule 100 fixée à l'os du patient.

L'action de l'utilisateur sur le levier pivotant 212 selon la flèche F1 a pour effet de faire exercer par la lame levier 211 une traction axiale selon la flèche F3 à l'arbre d'expansion 240 qui se déplace en translation selon cette flèche. Les surfaces inclinées périphériques extérieures 241 ménagées sur la surface extérieure dudit arbre d'expansion mobile 240 viennent alors exercer une contrainte radiale selon les flèches F4. Cette contrainte selon la flèche F4 se répercute sur les surfaces intérieures 232 et donc extérieures 231 des projections de la couronne d'agrippement 230 laquelle la répercute sur les lamelles 330 de l'interface mécanique 300 garantissant la préhension du module d'agrippement 220 sur l'interface mécanique 300 et de l'interface mécanique 300 sur la cupule 100.

Le retour en position de repos du levier pivotant 212 permet au module d'agrippement de ne plus exercer de contrainte sur l'interface mécanique 300 et de libérer son volume concave interne 320.

Le fait de laisser en place l'interface mécanique 300 dans la cupule 100 elle-même fixée à l'os du patient peut permettre la mise en oeuvre d'une autre fonction de l'invention.

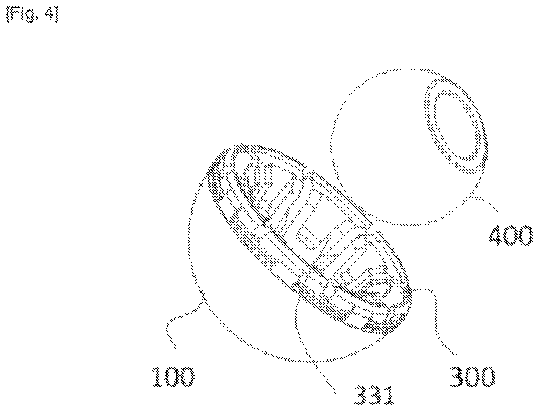

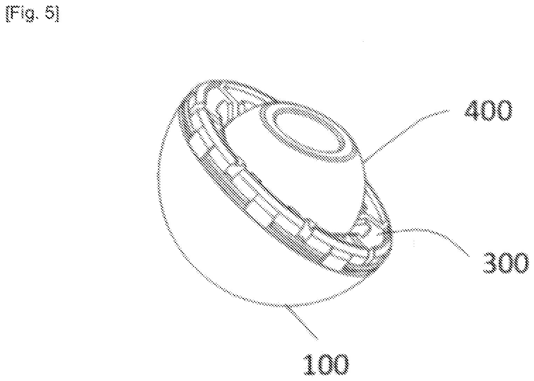

En effet, comme illustrée par les figures 4 et 5, l'interface mécanique 300 est conçue de façon à servir d'insert d'essai à des têtes d'essai ou définitives 400.

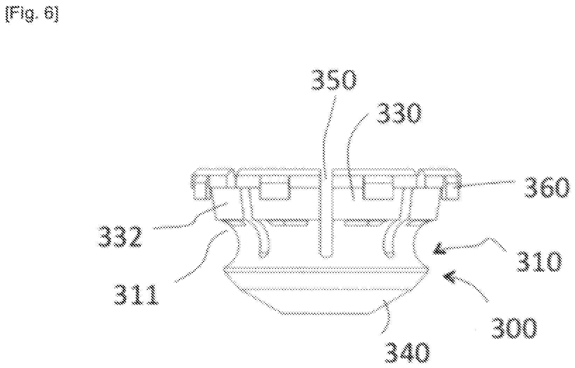

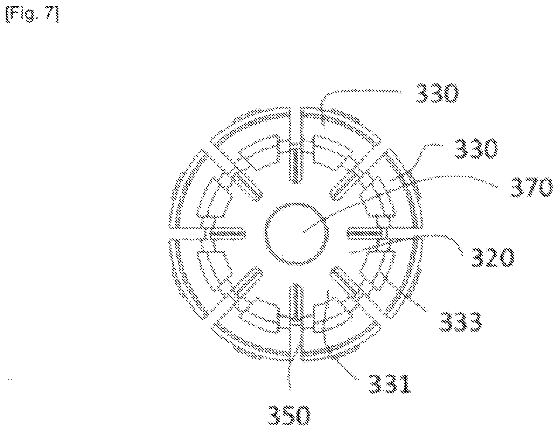

Pour ce faire comme illustré par les figures 6 et 7. le volume concave 320 de l'interface mécanique est dimensionné et préformé pour présenter des surfaces d'appui correspondant à celles de l'insert destiné à être accueilli après mise en place de la cupule 100. La surface interne du profil desdites lamelles 300 est été préformée de façon à former le volume d'accueil et de retenue pour la tête,

On comprend que le dispositif qui vient d'être ci-dessus décrit et représenté, l'a été en vue d'une divulgation plutôt que d'une limitation, Bien entendu, divers aménagements, modifications et améliorations pourront être apportés aux exemples ci-dessus, sans pour autant sortir du cadre de l'invention.

Ainsi, bien que la cupule, l'interface mécanique et la tête illustrées dans ce mode de réalisation non limitatif adoptent une configuration dite simple mobilité. l'invention s'applique également avec des formes et des dimensions adaptées à une configuration dite double mobilité.

## Revendications

1. Dispositif de préhension (D) pour élément prothétique (100) telle une cupule,
l'élément prothétique (100) comprenant un volume inférieur présentant une surface intérieure.
le dispositif comprenant un outil de préhension (O) coopérant ponctuellement avec l'élément prothétique (100) à des fins de manipulation et/ou d'installation sur le patient,
ledit outil comprenant un manche (200) avec à une extrémité, une poignée (210) et à une autre extrémité, un module d'agrippement (220),
ledit module d'agrippemert (220) étant commandé par l'action de l'utilisateur sur une lame levier (211) associée au manche pour passer d'une position de retenue à une position de repos et vice-versa,
ledit dispositif comprenant une interface mécanique (300) s'intercalant entre l'élément prothétique (100) et le module d'agrippement (220) et venant se fixer à l'élément prothétique (100),
ladite interface mécanique (300) présentant une surface extérieure convexe (310) se projetant en partie dans le volume intérieur de Tl'élément prothétique (100) et épousant la surface intérieure de l'élément prothétique (100), et un volume intérieur concave (320) avec lequel coopère le module d'agrippement (220),
l'interface mécanique (300) se composant d'une pluralité de lamelles (330) qui, rayonnant autour d'une même partie centrale, adoptent un profil avec une surface interne (320) et une surface externe (310), ladite surface externe (310) forme ladite surface extérieure convexe pour épouser tout ou partie du profil de la surface intérieure (110) de rélément prothétique (100),
ladite interface mécanique (300) étant préformée et réalisée dans un matériau tel que son élasticité permet sa retenue dans l'élément prothétique (100), de sorte que
- la position de repos du module d'agrippement (220) commandée par la lame levier (211) autorise ce dernier à venir s'introduire dans le volume intérieur concave (320) et à sortir de ce denier, et que
- la position de retenue permet au module d'agrippement (220) introduit dans le volume intérieur concave (320) d'agripper l'interface (300) et de retenir l'élément prothétique (100) lorsque ce dernier doit être déplacé ou de libérer l'interface mécanique (300) de l'élément prothétique (100) lorsque ce dernier est en place et fixé à l'os du patient,
le volume inférieur concave (320) de l'interface mécanique (300) étant préformé de surfaces périphériques d'appui (331) avec lesquelles coopèrent des surfaces de retenue périphériques préformées dans le modute d'agrippement (220), de sorte qu'un mouvement commandé par la lame levier (211) assure la création d'uns contrainte tendant à étendre les surfaces de retenue périphériques de façon à assurer la fixation ponctuelle du module d'agrippement (220) à l'interface mécanique (300), **caractérisé par le fait que**
ledit module d'agrippement (220) comprend une couronne d'agrippement (230) équipée extérieurement desdites surfaces de retenue périphériques (231) et intérieurement de surfaces inclinées périphériques intérieures (232) coopérant avec des surfaces inclinées périphériques extérieures (241) ménagées sur la surface extérieure d'un arbre d'expansion (240) mobile en translation et dont une extrémité est articulée à la lame levier (211) de sorte que l'action sur la lame levier (211) provoque un mouvement en translation amenant à l'expansion des surfaces de retenue périphériques (231) ou leur rétractation en position de repos.

2. Dispositif (D) selon la revendication 1, dans lequel la surface extérieure convexe (310) formée par la surface externe desdites lamelles (330), est préformée d'une gorge périphérique (311) ménagée entre une partie de surface convexe se positionnant au fond du volume intérieur de l'élément prothétique (100) et une partie de surface convexe se positionnant au niveau du rebord de l'élément prothétique (100),

3. Dispositif(D) selon la revendication 1, dans lequel la surface interne du profil desdites lamelles (330) est préformée desdites surfaces périphériques d'appui (331),

4. Dispositif(D) selon la revendication 1, dans lequel l'interface mécanique (300) comporte des dents (360) coopérant avec des surfaces préformées sur le rebord de la cupule (100),

5. Dispositif(D) selon la revendication 1, dans lequel le volume concave (320) de l'interface mécanique (300) est dimensionné et préformé pour présenter des surfaces d'appui correspondant à celles d'un insert destiné à être accueilli après mise en place de l'élément prothétique à des fins d'accueil d'une tête d'essai ou définitive (400).

6. Dispositif selon les revendications 1 et 5_{,} dans lequel la surface interne du profil desdites lamelles est préformée de façon à former le volume d'accueil pour l'insert.

7. Dispositif selon l'une quelconque des revendications précédentes, dans lequel le matériau de l'interface mécanique (300) est de la résine polypropyléne.

8. Dispositif selon la revendication 1 dans lequel ledit module d'agrippement (220) comprend une tête (250) du module d'agrippement (220) qui guide la translation de l'arbre d'expansion (240). ladite tête (250) étant fendue par une fente (251).

9. Dispositif selon la revendication 8, dans lequel ladite couronne d'agrippement (230) comprend une fente (234) ouvrant l'anneau qu'elle forme.

10. Procédé d'utilisation d'un dispositif de préhension pour élèment prothétique telle une cupule du dispositif selon l'une quelconque des revendications 1 à 9, **CARACTÉRISÉ EN CE QU'**il consiste à utiliser ladite interface mécanique (300) comme insert d'essai.

## Patentansprüche

1. Greifvorrichtung (D) für ein Prothesenelement (100), wie etwa eine Pfanne,
wobei das Prothesenelement (100) ein Innenvolumen umfasst, das eine Innenfläche aufweist,
wobei die Vorrichtung ein Greifwerkzeug (O) umfasst, welches an bestimmten Stellen mit dem Prothesenelement (100) zusammenarbeitet, um es zu handhaben und/oder am Patienten anzubringen;
wobei das Werkzeug einen Stiel (200) umfasst, der an einem Ende ein Handstück (210) und am anderen Ende ein Greifmodul (220) aufweist,
wobei das Greifmodul (220) dadurch gesteuert wird, dass der Bediener auf ein Hebelblatt (211) einwirkt, welches mit dem Stiel in Verbindung steht, um von einer Haltestellung in eine Ruhestellung überzugehen und umgekehrt,
wobei die Vorrichtung Folgendes umfasst
eine mechanische Schnittstelle (300), die zwischen dem Prothesenelement (100) und dem Greifmodul (220) eingeführt ist und die eine feste Verbindung mit dem Prothesenelement (100) eingeht,
wobei die mechanische Schnittstelle (300) eine konvexe Außenfläche (310), welche zum Teil in das Innenvolumen des Prothesenelements (100) ragt und an der Innenfläche des Prothesenelements (100) anliegt, und ein konkaves Innenvolumen (320) aufweist, mit welchem das Greifmodul (220) zusammenwirkt,
wobei die mechanische Schnittstelle (300) sich aus einer Mehrzahl an Lamellen (330) zusammensetzt, die sich ausgehend von demselben mittigen Abschnitt erstrecken und ein Profil mit einer inneren Fläche (320) und einer äußeren Fläche (310) annehmen, wobei die äußere Fläche (310) die konvexe Außenfläche derart bildet, dass sie vollständig oder teilweise am Profil der Innenfläche (110) des Prothesenelements (100) anliegt,
wobei die mechanische Schnittstelle (300) vorgeformt ist und aus einem Material hergestellt ist, dessen Elastizität es ermöglicht, sie im Prothesenelement (100) zu halten,
derart, dass
- das Greifmodul (220), welches durch das Hebelblatt (211) gesteuert wird, in seiner Ruhestellung in das konkave Innenvolumen (320) eintreten und dieses verlassen kann, und dass
- das Greifmodul (220), wenn es in das konkave Innenvolumen (320) eingesetzt ist, in seiner Haltestellung die Schnittstelle (300) ergreifen und das Prothesenelement (100) festhalten kann, wenn letzteres bewegt werden soll, oder die mechanische Schnittstelle (300) des Prothesenelements (100) freigeben kann, wenn dieses sich an Ort und Stelle befindet und am Knochen des Patienten befestigt ist,
wobei das konkave Innenvolumen (320) der mechanischen Schnittstelle (300) derart vorgeformt ist, dass es randständige Auflageflächen (331) aufweist, mit welchen vorgeformte randständige Halteflächen im Greifmodul (220) zusammenwirken, sodass eine Bewegung, welche durch das Hebelblatt (211) gesteuert wird, sicherstellt, dass eine Spannung erzeugt wird, die dazu neigt, die randständigen Halteflächen derart auszudehnen, dass sichergestellt wird, dass das Greifmodul (220) an bestimmten Stellen eine feste Verbindung mit der mechanischen Schnittstelle (300) eingeht,
**DADURCH GEKENNZEICHNET, DASS**
das Greifmodul (220) einen Greifkranz (230) aufweist, der außen mit den randständigen Halteflächen (231) und innen mit inneren randständigen Schrägflächen (232) versehen ist, die mit äußeren randständigen Schrägflächen (241) zusammenwirken, welche an der Außenfläche einer Aufweitungswelle (240) vorgesehen sind, welche eine Translationsbewegung ausführen kann, wobei eines ihrer Enden derart an das Hebelblatt (211) angelenkt ist, dass ein Einwirken auf das Hebelblatt (211) eine Translationsbewegung verursacht, welche zu einer Aufweitung der randständigen Halteflächen (231) oder zu deren Rückzug in die Ruhestellung führt.

2. Vorrichtung (D) nach Anspruch 1, wobei die konvexe Außenfläche (310), welche von der äußeren Fläche der Lamellen (330) gebildet wird, aus einer randständigen Nut (311) vorgeformt ist, die zwischen einem konvexen Flächenabschnitt, welcher sich am Boden des Innenvolumens des Prothesenelements (100) befindet, und einem konvexen Flächenabschnitt vorgesehen ist, welcher sich an der Kante des Prothesenelements (100) befindet.

3. Vorrichtung (D) nach Anspruch 1, wobei die inneren Fläche des Profils der Lamellen (330) aus den randständigen Auflageflächen (331) vorgeformt ist.

4. Vorrichtung (D) nach Anspruch 1, wobei die mechanische Schnittstelle (300) Zähne (360) aufweist, die mit vorgeformten Flächen an der Kante der Pfanne (100) zusammenwirken.

5. Vorrichtung (D) nach Anspruch 1, wobei das konkave Volumen (320) der mechanischen Schnittstelle (300) derart bemessen und vorgeformt ist, dass es Auflageflächen aufweist, die denjenigen einer Einlage entsprechen, welche dazu bestimmt ist, aufgenommen zu werden, nachdem das Prothesenelement eingesetzt wurde, um einen Versuchskopf oder endgültigen Kopf (400) aufzunehmen.

6. Vorrichtung nach den Ansprüchen 1 und 5, wobei die innere Fläche des Profils der Lamellen derart vorgeformt ist, dass sie das Aufnahmevolumen für die Einlage bildet.

7. Vorrichtung nach einem beliebigen der vorhergehenden Ansprüche, wobei es sich bei dem Material der mechanischen Schnittstelle (300) um Polypropylenharz handelt.

8. Vorrichtung nach Anspruch 1, wobei das Greifmodul (220) einen Kopf (250) des Greifmoduls (220) umfasst, der die Translation der Aufweitungswelle (240) führt, wobei der Kopf (250) von einen Schlitz (251) gespalten wird.

9. Vorrichtung nach Anspruch 8, wobei der Greifkranz (230) einen Schlitz (234) umfasst, der den Ring öffnet, welchen er bildet.

10. Verfahren zur Verwendung einer Greifvorrichtung für ein Prothesenelement wie etwa eine Pfanne der Vorrichtung nach einem beliebigen der Ansprüche 1 bis 9, **DADURCH GEKENNZEICHNET, DASS** es darin besteht, die mechanische Schnittstelle (300) als versuchsweise Einlage zu verwenden.

## Claims

1. Gripping device (D) for a prosthetic element (100) such as a cup,
the prosthetic element (100) comprising an inner volume having an inner surface,
the device comprising a gripping tool (O) engaging occasionally with the prosthetic element (100) for the purpose of handling and/or fitting on the patient,
said tool comprising a shaft (200) with, at one end, a handle (210) and, at another end, a grip module (220),
said grip module (220) being controlled by the action of the user on a lever blade (211) associated with the shaft to switch from a retaining position to a resting position and vice versa,
said device comprising
a mechanical interface (300) interleaved between the prosthetic element (100) and the grip module (220) and fastened to the prosthetic element (100),
said mechanical interface (300) having a convex outer surface (310) projecting partially into the inner volume of the prosthetic element (100) and moulding the inner surface of the prosthetic element (100), and a concave inner volume (320) with which the grip module (220) engages,
the mechanical interface (300) consisting of a plurality of strips (330) which, radiating around one same central part, adopt a profile with an inner surface (320) and an outer surface (310), said outer surface (310) forms said convex outer surface to mould all or part of the profile of the inner surface (110) of the prosthetic element (100),
said mechanical interface (300) being preformed and made of a material such that its elasticity enables it to be retained in the prosthetic element (100),
such that
- the rest position of the grip module (220) controlled by the lever blade (211) enables the latter to be introduced into the concave inner volume (320) and to exit the latter, and that
- the retaining position enables the grip module (220) introduced into the concave inner volume (320) to grip the interface (300) and to retain the prosthetic element (100) when the latter must be moved or to release the mechanical interface (300) from the prosthetic element (100) when the latter is in place and fastened to the patient's bone, the concave inner volume (320) of the mechanical interface (300) being preformed with peripheral bearing surfaces (331) with which peripheral retaining surfaces preformed in the grip module (220) engage, such that a movement controlled by the lever blade (211) creates a stress tending to extend the peripheral retaining surfaces, so as to ensure the occasional fastening of the grip module (220) to the mechanical interface (300),
**CHARACTERISED IN THAT**
said grip module (220) comprises a grip crown (230) equipped externally with said peripheral retaining surfaces (231) and internally with inner peripheral inclined surfaces (232) engaging with outer peripheral inclined surfaces (241) provided on the outer surface of an expansion shaft (240) movable in translation and one end of which is articulated to the lever blade (211), such that the action on the lever blade (211) causes a translational movement leading to the expansion of the peripheral retaining surfaces (231) or their retraction into the rest position.

2. Device (D) according to claim 1, wherein the convex outer surface (310) formed by the outer surface of said strips (330), is preformed with a peripheral groove (311) provided between a convex surface part positioned at the bottom of the inner volume of the prosthetic element (100) and a convex surface part positioned at the rim of the prosthetic element (100).

3. Device (D) according to claim 1, wherein the internal surface of the profile of said strips (330) is preformed from said peripheral bearing surfaces (331).

4. Device (D) according to claim 1, wherein the mechanical interface (300) comprises teeth (360) engaging with surfaces preformed on the rim of the cup (100).

5. Device (D) according to claim 1, wherein the concave volume (320) of the mechanical interface (300) is sized and preformed to have bearing surfaces corresponding to those of an insert intended to be accommodated after placement of the prosthetic element for the purpose of accommodating a test or final head (400).

6. Device according to claims 1 and 5, wherein the internal surface of the profile of said strips is preformed so as to form the accommodating volume for the insert.

7. Device according to any one of the preceding claims, wherein the material of the mechanical interface (300) is polypropylene resin.

8. Device according to claim 1, wherein said grip module (220) comprises a head (250) of the grip module (220) which guides the translation of the expansion shaft (240), said head (250) being slit by a slot (251).

9. Device according to claim 8, wherein said grip crown (230) comprises a slot (234) opening the ring that it forms.

10. Method for using a gripping device for a prosthetic element such as a cup of the device according to any one of claims 1 to 9, **CHARACTERISED IN THAT** it consists of using said mechanical interface (300) as a test insert.
